(19) Europäisches Patentamt
European Patent Office
Office européen des brevets

(11) **EP 4 492 042 A1**

(12) **EUROPEAN PATENT APPLICATION**

(43) Date of publication:
**15.01.2025 Bulletin 2025/03**

(21) Application number: **23184736.9**

(22) Date of filing: **11.07.2023**

(51) International Patent Classification (IPC):
**G01N 21/47** (2006.01)  **G01N 21/31** (2006.01)
**A61B 5/00** (2006.01)  **G01J 3/02** (2006.01)
**G01N 21/27** (2006.01)

(52) Cooperative Patent Classification (CPC):
**G01N 21/31; G01J 3/02; G01J 3/10; G01N 21/474;**
A61B 5/0075; A61B 2560/0233; G01N 21/274;
G01N 2021/4711; G01N 2021/4733;
G01N 2021/4735

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **trinamiX GmbH
67063 Ludwigshafen am Rhein (DE)**

(72) Inventors:
• **Steeg, Matthias
  67063 Ludwigshafen am Rhein (DE)**
• **Schmidt, Felix
  67059 Ludwigshafen am Rhein (DE)**

(74) Representative: **Altmann Stößel Dick
Patentanwälte PartG mbB
Theodor-Heuss-Anlage 2
68165 Mannheim (DE)**

(54) **SURFACE CHARACTERIZATION VIA AN ANGLE-DEPENDENT SPECTRAL RESPONSE**

(57)  A spectrometer device (110) for determining an item of spectral information on at least one object (112) is described, the spectrometer device (110) comprising:
- at least one sample interface (130), wherein the sample interface (130) is designated to define a measurement pose of the object (112);
- at least one light emitting element (116), wherein the at least one light emitting element (116) is designated to emit illumination light (118) onto the object (112) for generating detection light (119);
- at least two photosensitive detectors (122) designated to generate at least one detector signal when receiving detection light (119), wherein a first angle of incidence β of detection light (119) that is incident on the sample interface (130) and propagating to a first photosensitive detector (126) of the at least two photosensitive detectors (122) is different from a second angle of incidence β of detection light (119) that is incident on the sample interface (130) and propagating to a second photosensitive detector (128) of the at least two photosensitive detectors (122), wherein the first photosensitive detector (126) and the second photosensitive detector (128) detect light of the same spectral range of the detection light (119);
- at least one evaluation unit (140), wherein the at least one evaluation unit (140) is configured to consider the detector signals of the at least two photosensitive detectors (122) for determining an item of reflectance information on the object (112).

FIG.2

## Description

Technical Field

The invention relates to a spectrometer device for determining an item of spectral information on at least one object and a method for determining reflectance information on the object. The invention further relates to a computer program, a computer-readable storage medium and to a non-transient computer-readable medium. Such devices and methods can, in general, be used for investigating or monitoring purposes, in particular, in the infrared (IR) spectral region, especially in the near-infrared (NIR) spectral region, and in the visible (VIS) spectral region, e.g. in a spectral region allowing to mimic a human's ability of color sight. However, further applications are feasible.

Background art

Spectrometer devices and other measurement systems based on electromagnetic radiation, specifically waves, provide information determined by considering a spectral response, i.e. at least one signal level related to the reflectance, transmittance and/or absorbance of an object, that is compared to a calibration measurement.

Depending on the optical path design, the spectral response of the spectrometer device may be dependent on the angle of incidence. This dependency particularly affects spectrometer devices having a folded optical designs and/or that comprise angle dependent optical components, such as reflective filters. Based on the optical design of the spectrometer, different spectral components may have at least one different optical path, e.g. due to different positions of detectors. These spectral components may exhibit a different dependence on the angle of incidence and a different acceptable angular range.

Objects of the same material may thus exhibit different properties, e.g. surface roughness, that may affect the scattering properties of the sample, but not the molecular absorption of the material itself. External and/or internal conditions may alter the scattering properties, specifically of biological samples. Based on different manufacturing techniques and/or due to different conditions the properties of an object, especially on its surface, such as a roughness and the like, may vary. This, particularly, may affects the scattering properties of the object. The scattering properties of the object affect the angle of incidence of the collected sample light, which further affects the spectral response of spectrometer device showing a dependence on the angle of incidence.

Thus, when the angle-of-incidence is not taken into account scattering properties of the sample can negatively affect the obtained spectroscopic information. In order for the scattering properties to not negatively affect the performance of a spectrometer device, they need to be taken into account. By considering the angle of incidence of the object, additional information may be obtained.

Problem to be solved

It is therefore desirable to provide methods and devices, which at least partially address the above-mentioned technical challenges and at least substantially avoid the disadvantages of known methods and devices. In particular, it is an object of the present invention to provide a spectrometer device which is capable of determining and/or accounting for the scattering properties and/or scattering proportions of a sample.

Summary

This problem is addressed by a spectrometer device for determining an item of spectral information on at least one object, a method for determining reflectance information on the object, a computer program, a computer-readable storage medium and to a non-transient computer-readable medium, with the features of the independent claims. Advantageous embodiments which might be realized in an isolated fashion or in any arbitrary combinations are listed in the dependent claims as well as throughout the specification.

In a first aspect, a spectrometer device for determining an item of spectral information on at least one object is disclosed.

The term "spectrometer device" as used herein is a broad term and is to be given its ordinary and customary meaning to a person of ordinary skill in the art and is not to be limited to a special or customized meaning. The term specifically may refer, without limitation, to an optical device configured for acquiring at least one item of spectral information on at least one object. Specifically, the at least one item of spectral information may refer to at least one optical property or optically measurable property which is determined as a function of a wavelength, for one or more different wavelengths. More specifically, the optical property or optically measurable property, as well as the at least one item of spectral information, may relate to at least one property characterizing at least one of a transmission, an absorption, a reflection and an emission of the at least one object, either by itself or after illumination with external light. The at least one optical property may be determined for one or more wavelengths. The spectrometer device specifically may form an apparatus which is capable of recording a signal intensity with respect to the corresponding wavelength of a spectrum or a partition thereof, such as a wavelength interval, wherein the signal intensity may, specifically, be provided as an electrical signal which may be used for further evaluation.

The term "spectroscopic information", also referred to as "spectral information" or as "an item of spectral information", as used herein is a broad term and is to be given its ordinary and customary meaning to a person of ordinary skill in the art and is not to be limited to a special or customized meaning. The term specifically may refer, without limitation, to an item of information, e.g. on at least one object and/or radiation emitted by at least one object, characterizing at least one optical property of the object, more specifically at least one item of information characterizing, e.g. qualifying and/or quantifying, at least one of a transmission, an absorption, a reflection and an emission of the at least one object. As an example, the at least one item of spectral information may comprise at least one intensity information, e.g. information on an intensity of light being at least one of transmitted, absorbed, reflected or emitted by the object, e.g. as a function of a wavelength or wavelength sub-range over one or more wavelengths, e.g. over a range of wavelengths. Specifically, the intensity information may correspond to or be derived from the signal intensity, specifically the electrical signal, recorded by the spectrometer device with respect to a wavelength or a range of wavelengths of the spectrum.

The term "object" as used herein is a broad term and is to be given its ordinary and customary meaning to a person of ordinary skill in the art and is not to be limited to a special or customized meaning. The term specifically may refer, without limitation, to an arbitrary body, chosen from a living object and a non-living object. Thus, as an example, the at least one object may comprise one or more articles and/or one or more parts of an article, wherein the at least one article or the at least one part thereof may comprise at least one component which may provide a spectrum suitable for investigations. Additionally or alternatively, the object may be or may comprise one or more living beings and/or one or more parts thereof, such as one or more body parts of a human being, e.g. a user, and/or an animal. The object specifically may comprise at least one sample which may fully or partially be analyzed by spectroscopic methods. As an example, the object may be or may comprise at least one of: human or animal skin; edibles, such as fruits; plastics and textile.

The spectrometer device comprises:

- at least one sample interface, wherein the sample interface is designated to define a measurement pose of the object;
- at least one light emitting element, wherein the at least one light emitting element is designated to emit illumination light onto the object for generating detection light;
- at least two photosensitive detectors designated to generate at least one detector signal when receiving detection light, wherein a first angle of incidence • of detection light that is incident on the sample interface and propagating to a first photosensitive detector of the at least two photosensitive detectors is different from a second angle of incidence $\beta$ of detection light that is incident on the sample interface and propagating to a second photosensitive detector of the at least two photosensitive detectors, wherein the first photosensitive detector and the second photosensitive detector detect light of the same spectral range of the detection light;
- at least one evaluation unit, wherein the at least one evaluation unit is configured to consider the detector signals of the at least two photosensitive detectors for determining an item of reflectance information on the object.

The spectrometer device comprises at least one sample interface, wherein the sample interface is designated to define a measurement pose of the object.

The term "sample interface" as used herein is a broad term and is to be given its ordinary and customary meaning to a person of ordinary skill in the art and is not to be limited to a special or customized meaning. The term specifically may refer, without limitation, to a predefined surface, specifically a plane, between a sample and a measurement tool. The sample interface may be a surface and/or plane at which the light emitted by the spectrometer device interacts with the object in a manner that detection light is generated. The sample interface may act as an aperture and/or an opening. Particularly for carrying out optimal measurement, the sample interface may define an intended measurement position and/or an intended measurement orientation for the object during the measurements. When performing at least one measurement, particularly for determining the item of spectral information, the object has to be placed and/or oriented correctly in regard to the sample interface for performing accurate and reliable measurements. The sample interface may define an intended placement for the object position and/or the object orientation in a manner that the optical path from the light emitting element via the object on a respective detector follows a predetermined and/or intended path.

The sample interface may be a measurement window, particularly comprising silicone, designated for transmitting the illumination light and the detection light. The term "measurement window" as used herein is a broad term and is to be given its ordinary and customary meaning to a person of ordinary skill in the art and is not to be limited to a special or customized meaning. The term specifically may refer, without limitation, to a contact surface and/or lay-on-surface for the object to be investigated, particularly thereby defining the predetermined position and/or orientation of the object. The object to be investigated may be placed against the measurement window before the process of measuring the spectroscopic information may be started. During the measurement process the object may remain against the measurement window, particularly until the measurement process may be completed. Thereby, the measurement conditions, specifically a distance and/or an orientation, but also a position, of the object in relation to the spectrometer device, specifically the sample interface, may be defined. The measurement window may allow the illumination light and/or detection light to transfer. The measurement window may be transparent to the illumination light and/or detection light.

The term "pose" as used herein is a broad term and is to be given its ordinary and customary meaning to a person of ordinary skill in the art and is not to be limited to a special or customized meaning. The term specifically may refer, without limitation, to an orientation and/or a position of a rigid body. A pose may be described by using six components. For describing a position, three components may be used, such as coordinates associated with a center of mass of the rigid body in three-dimensional space. For describing an orientation, three components may be used, typically the Euler angles. Together, these components may describe the full pose of the rigid body. In accordance with the present invention, the pose of the object for during a measurement may be predetermined in a manner that a position and/or an orientation of the object, particularly in relation to the spectrometer device, specifically the sample interface, is defined. Particularly thereby, at least one optical path, preferably a plurality of optical path, even more preferably any optical path, from the light emitting element onto the object and/or from the object onto the at least two photosensitive detectors is optimized, and particularly may compare, thereby, compare to an intended optical path.

The spectrometer device comprises at least one light emitting element, wherein the at least one light emitting element is designated to emit illumination light onto the object for generating detection light.

As further used herein, the term "light" as used herein is a broad term and is to be given its ordinary and customary meaning to a person of ordinary skill in the art and is not to be limited to a special or customized meaning. The term specifically may refer, without limitation, to electromagnetic radiation in one or more of the infrared, the visible and the ultraviolet spectral range. Herein, the term "ultraviolet spectral range", generally, refers to electromagnetic radiation having a wavelength of 1 nm to 380 nm, preferably of 100 nm to 380 nm. Further, in partial accordance with standard ISO-21348 in a valid version at the date of this document, the term "visible spectral range", generally, refers to a spectral range of 380 nm to 760 nm. The term "infrared spectral range" (IR) generally refers to electromagnetic radiation of 760 nm to 1000 $\mu$m, wherein the range of 760 nm to 1.5 $\mu$m is usually denominated as "near infrared spectral range" (NIR) while the range from 1.5 $\mu$ to 15 $\mu$m is denoted as "mid infrared spectral range" (MidiR) and the range from 15 $\mu$m to 1000 $\mu$m as "far infrared spectral range" (FIR). Preferably, light used for the typical purposes of the present invention is light in the infrared (IR) spectral range, more preferred, in the near infrared (NIR) and/or the mid infrared spectral range (MidiR), especially the light having a wavelength of 1 $\mu$m to 5 $\mu$m, preferably of 1 $\mu$m to 3 $\mu$m. This is due to the fact that many material properties or properties on the chemical constitution of many objects may be derived from the near infrared spectral range. It shall be noted, however, that spectroscopy in other spectral ranges is also feasible and within the scope of the present invention.

Consequently, the term "light emitting element", also referred to as an "illumination source", as used herein is a broad term and is to be given its ordinary and customary meaning to a person of ordinary skill in the art and is not to be limited to a special or customized meaning. The term specifically may refer, without limitation, to an arbitrary device configured for generating or providing light, specifically "illumination light" in the sense of the above-mentioned definition for the term "light". The radiation emitting element specifically may be or may comprise at least one electrical light source, such as an electrically driven light source.

As further used herein, the term "detection light" as used herein is a broad term and is to be given its ordinary and customary meaning to a person of ordinary skill in the art and is not to be limited to a special or customized meaning. The term specifically may refer, without limitation, to light that is generated by the object, particularly generated in an interaction of the illumination light with the object, such as scattering, reflecting and/or transmitting. The detection light may be illumination light that is reflected and/or scattered back through the sample interface to the photosensitive detectors. At least a portion of the illumination light may be transmitted and/or absorbed by the object in a manner that it is not detected by the photosensitive detectors.

The radiation emitting element may be a thermal radiator. The thermal radiator may be selected from an incandescent lamp or a thermal infrared emitter. The term "incandescent lamp" as used herein is a broad term and is to be given its ordinary and customary meaning to a person of ordinary skill in the art and is not to be limited to a special or customized meaning. The term specifically may refer, without limitation, to an electric light having a heatable element, such as a wire filament heated, which is capable of being heated to a temperature at which it emits light, especially infrared light. Since the incandescent lamp can, therefore, be considered as a thermal emitter within the infrared spectral range, an emission power of the incandescent lamp decreases with increasing wavelength. The thermal radiator may be selected from an incandescent lamp or a thermal infrared emitter. The term "thermal infrared emitter" as used herein is a broad term and is to be given its ordinary and customary meaning to a person of ordinary skill in the art and is not to be limited to a special or customized meaning. The term specifically may refer, without limitation, to a micro-machined thermally emitting device, which comprises a radiation emitting surface as the radiation emitting element that emits the optical radiation to be monitored.

Alternatively or in addition, the radiation emitting element may be a laser, specifically a vertical cavity surface emitting laser (VCSEL), particularly emitting at least one wavelength in the infrared region.

The term "vertical-cavity surface-emitting laser" as used herein is a broad term and is to be given its ordinary and customary meaning to a person of ordinary skill in the art and is not to be limited to a special or customized meaning. The term specifically may refer, without limitation, to a semiconductor laser diode configured for laser beam emission perpendicular with respect to a top surface. VCSELs are generally known to the skilled person such as from WO 2017/222618 A.

Alternatively or in addition, the radiation emitting element may be a light-emitting diode (LED), specifically a LED emitting light that is at least partially located in the infrared spectral range. Alternatively or in addition, a LED emitting light that is illuminating a luminescent material, specifically a phosphor, for light-conversion of light generated by the LED, wherein the luminescent material generates converted light that is at least partly located in the near-infrared spectral range.

The term "light-emitting diode" or briefly "LED", as used herein, is a broad term and is to be given its ordinary and customary meaning to a person of ordinary skill in the art and is not to be limited to a special or customized meaning. The term specifically may refer, without limitation, to an optoelectronic semiconductor device capable of emitting light when an electrical current flows through the device. The optoelectronic semiconductor device may be configured for generating the light due to various physical processes, including one or more of spontaneous emission, induced emission, decay of metastable excited states and the like. Thus, as an example, the light-emitting diode, may comprise one or more of: a light-emitting diode based on spontaneous emission of light, in particular an organic light emitting diode, a light-emitting diode based on superluminescence (sLED), or a laser diode (LD) In the following, without narrowing the possible embodiments of the light-emitting diode to any of the before-mentioned physical principles or setups, the abbreviation "LED" will be used for any type of light-emitting diode.

Specifically, the LED may comprise at least two layers of semiconductor material, wherein light may be generated at at least one interface between the at least two layers of semiconductor material, specifically due to a recombination of positive and negative electrical charges, e.g. due to electron-hole recombination. The at least two layers of semiconductor material may have differing electrical properties, such as at least one of the layers being an n-doped semiconductor material and at least one of the layers being a p-doped semiconductor material. Thus, as an example, the LED may comprise at least one pn-junction and/or at least one pin-set up. It shall be noted, however, that other device structures are feasible, too. The at least one semiconductor material may specifically be or may comprise at least one inorganic semiconducting material. It shall be noted, however, that organic semiconducting materials may be used additionally or alternatively.

Generally, the LED may convert electrical current into light, specifically light that is at least partially located in the infrared spectral range. Alternatively or in addition, LED may convert electrical current into light into primary light, more specifically into blue primary light. The LED, thus, specifically may be a blue LED. The LED may be configured for generating the primary light, particularly for the light-conversion in the phosphor, also referred to as the "pump light". Thus, the LED may also be referred to as the "pump LED". The LED specifically may comprise at least one LED chip and/or at least one LED die. Thus, the semiconductor element of the LED may comprise an LED bare chip.

The term "luminescence" as used herein is a broad term and is to be given its ordinary and customary meaning to a person of ordinary skill in the art and is not to be limited to a special or customized meaning. The term specifically may refer, without limitation, to the process of spontaneous emission of light by a substance not resulting from heat. Specifically, luminescence may refer to a cold-body radiation. More specifically, the luminescence may be initiated or excited by irradiation of light, in which case the luminescence is also referred to as "photoluminescence". The property of a material being capable of performing luminescence, in the context of the present invention, is referred to by the adjective "luminescent". The at least one luminescent material specifically may be a photoluminescent material, i.e. a material which is capable of emitting light after absorption of photons or excitation light. Specifically, the luminescent material may have a positive Stokes shift, which generally may refer to the fact that the secondary light is red-shifted with respect to the primary light.

The at least one luminescent material, thus, may form at least one converter, also referred to as a light converter, transforming primary light into secondary light having different spectral properties as compared to the primary light. Specifically, a spectral width of the secondary light may be larger than a spectral width of the primary light, and/or a center of emission of the secondary light may be shifted, specifically red-shifted, compared to the primary light. Specifically, the at least one luminescent material may have an absorption in the ultraviolet and/or blue spectral range and an emission in the near-infrared and/or infrared spectral range. Thus, generally, the luminescent material or converter may form at least one component of the phosphor LED converging primary light or pump light, specifically in the blue spectral range, into light having a longer wavelength, e.g. in the near-infrared or infrared spectral range.

The luminescent material, specifically, may, thus, form at least one converter or light converter. The luminescent material may form at least one of a converter platelet, a luminescent and specifically a fluorescent coating on the LED and phosphor coating on the LED. The luminescent material may, as an example, comprise one or more of the following materials: Cerium-doped YAG (YAG:$Ce^{3+}$, or $Y_3Al_5O_{12}$:$Ce^{3+}$); rare-earth-doped Sialons; copper- and aluminium-doped zinc sulfide (ZnS:Cu,Al).

The LED and the luminescent material, together, may form a so-called "phosphor LED". Consequently, the term "phosphor light-emitting diode" or briefly "phosphor LED", as used herein, is a broad term and is to be given its ordinary and customary meaning to a person of ordinary skill in the art and is not to be limited to a special or customized meaning. The term specifically may refer, without limitation, to a combination of at least one light-emitting diode configured for generating primary light or pump light, and at least one luminescent material, also referred to as a "phosphor", configured for light-conversion of the primary light generated by the light-emitting diode. The phosphor LED may form a packaged LED light source, including the LED die, e.g. a blue LED emitting blue pump light, as well as the phosphor, which, as an example, fully or partially coats the LED, which is, as an example, configured for converting the primary light or blue light into light having differing spectral properties, specifically into near-infrared light. Generally, the phosphor LED may be packaged in one housing or may be unpackaged. Thus, the LED and the at least one luminescent material for light-conversion of the primary light generated by the light-emitting diode may specifically be housed in a common housing. Alternatively, however, the LED may also be an unhoused or bare LED which may fully or partially be covered with the luminescent material, such as by disposing one or more layers of the luminescent material on the LED die. The phosphor LED, generally, may form an emitter or light source by itself.

The at least one luminescent material specifically may form at least one layer. Generally, various alternatives of positioning the luminescent material with respect to the light-emitting diode are feasible, alone or in combination. Firstly, the luminescent material, e.g., at least one layer of the luminescent material, such as the phosphor, may be positioned directly on the light-emitting diode, which is also referred to as a "direct attach", e.g. with no material in between the LED and the luminescent material or with one or more transparent materials in between, such as with one or more transparent materials, specifically transparent for the primary light, in between the LED and the luminescent material. Thus, as an example, a coating of the luminescent material may be placed directly or indirectly on the LED. Additionally or alternatively, the luminescent material, as an example, may form at least one converter body, such as at least one converter disk, which may be placed on top of the LED, e.g. by adhesive attachment of the converter body to the LED. Additionally or alternatively, the luminescent material may also be placed in a remote fashion, such that the primary light from the LED has to pass an intermediate optical path before reaching the luminescent material. This placement may also be referred to as a "remote placement" or as a "remote phosphor". Again, as an example, the luminescent material in the remote placement may form a solid body or converter body, such as a disk or converter disk. Further, in case of the remote placement, the luminescent material may also be a coating. In particular, an object which is transmitting light, e.g. a thin glass substrate, module window, comprising and/or being made of glass or plastics, may be coated with the phosphor. Alternatively, a reflective surface may be coated with the phosphor. This could be a flat or rough mirror, which may comprise and/or be made of a high-reflective index material substrate, e.g. silicon, or a gold, silver, aluminum or chromium coated flat or rough surface, e.g. glass, or a plastic. In the intermediate optical path, one or more optical elements may be placed, such as one or more of a lens, a prism, a grating, a mirror, an aperture or a combination thereof. Thus, specifically, an optical system having imaging properties may be placed in between the LED and the luminescent material, in the intermediate optical path. Thereby, as an example, the primary light may be focused, or bundled onto the converter body.

The spectrometer device comprises at least two photosensitive detectors designated to generate at least one detector signal when receiving detection light, wherein a first angle of incidence $\beta$ of detection light that is incident on the sample interface and propagating to a first photosensitive detector of the at least two photosensitive detectors is different from a second angle of incidence $\beta$ of detection light that is incident on the sample interface and propagating to a second photosensitive detector of the at least two photosensitive detectors, wherein the first photosensitive detector and the second photosensitive detector detect light of the same spectral range of the detection light.

The verb "to detect" as used herein is a broad term and is to be given its ordinary and customary meaning to a person of ordinary skill in the art and is not to be limited to a special or customized meaning. The term specifically may refer, without limitation, to the process of at least one of determining, measuring and monitoring at least one parameter, qualitatively and/or quantitatively, such as at least one of a physical parameter, a chemical parameter and a biological parameter. Specifically, the physical parameter may be or may comprise an electrical parameter. Consequently, the term "photosensitive detector" as used herein is a broad term and is to be given its ordinary and customary meaning to a person of ordinary skill in the art and is not to be limited to a special or customized meaning. The term specifically may refer, without limitation, to an arbitrary device configured for detecting, i.e. for at least one of determining, measuring and monitoring, at least one parameter, qualitatively and/or quantitatively, such as at least one of a physical parameter, a chemical parameter and a biological parameter. The photosensitive detectors may be configured for generating at least one detector signal, more specifically at least one electrical detector signal, such as an analogue and/or a digital detector signal, the detector signal providing information on the at least one parameter measured by the detector. The detector signal may directly or indirectly be provided by the photosensitive detectors to the evaluation unit, such that the photosensitive detectors and the evaluation unit may be directly or indirectly connected. The detector signals may be used as a "raw" detector signal and/or may be processed or preprocessed before further used, e.g. by filtering and the like. Thus, the photosensitive detectors may comprise at least one processing device and/or at least one preprocessing device, such as at least one of an amplifier, an analogue/digital converter, an electrical filter and a Fourier transformation.

The photosensitive detectors are configured for detecting light propagating from the object to the spectrometer device or more specifically to the photosensitive detectors of the spectrometer device. The photosensitive detectors may be configured for determining at least one optical parameter, such as an intensity and/or a power of light by which at least one sensitive area of the detector is irradiated. More specifically, the photosensitive detectors may comprise at least one photosensitive element and/or at least one optical sensor, such as at least one of a photodiode, a photocell, a photosensitive resistor, a phototransistor, a thermophile sensor, a photoacoustic sensor, a pyroelectric sensor, a photomultiplier and a bolometer. The photosensitive detectors, thus, may be configured for generating at least one detector signal, more specifically at least one electrical detector signal, in the above-mentioned sense, providing information on at least one optical parameter, such as the power and/or intensity of light by which the detector or a sensitive area of the detector is illuminated.

The photosensitive detectors may be comprised by at least one detector array, more specifically an array of photosensitive detectors. Each of the photosensitive detectors may comprise at least a photosensitive area which may be adapted for generating an electrical signal depending on the intensity of the incident light, wherein the electrical signal may, in particular, be provided to the evaluation unit, as will be outlined in further detail below. The photosensitive area as comprised by each of the optically sensitive photosensitive detectors may, especially, be a single, uniform photosensitive area which is configured for receiving the incident light which impinges on the individual optically sensitive elements. However, other arrangements of the optically sensitive elements may also be conceivable.

The at least two photosensitive detectors may be designated to receive detection light reflected and/or scattered by the object. For being designated to receive detection light reflected and/or scattered by the object, the at least two photosensitive detectors may be arranged and/or positioned in the spectrometer device such that light that is emitted by the emitting element is incident on the object, particularly when the object is placed accordingly. The object may then generate light, such as by reflecting and/or scattering the incident light. Reflection may typically occur, when the surface of the object is smooth. Scattering may typically occur when the surface of the object is rough and/or comprises irregularities. The spectrum of the scattered light and/or the reflected light may be influenced by the interaction of the light with the object.

As used herein, the term "angle of incidence $\beta$" is a broad term and is to be given its ordinary and customary meaning to a person of ordinary skill in the art and is not to be limited to a special or customized meaning. The term specifically may refer, without limitation, to an angle between the line of sight of a respective photosensitive detector and a normal vector of a surface of the sample interface.

Particularly for detecting light of the same spectral range of the detection light, the spectrometer device may comprise at least one filter element arranged in a beam path of the detection light. As used herein, the term "filter element" is a broad term and is to be given its ordinary and customary meaning to a person of ordinary skill in the art and is not to be limited to a special or customized meaning. The term specifically may refer, without limitation, to an arbitrary optical element which interacts with differing spectral portions of incident light in a different manner, e.g. by having at least one wavelength-dependent optical property, such as at least one wavelength-dependent optical property selected from the list consisting of a degree of reflection, a direction of reflection, a degree of refraction, a direction of refraction, an absorption, a transmission, an index of refraction.

The filter element may be configured such that each of the photosensitive detectors may be exposed to the same spectral range of the detection light. The filter element may be selected from the group of a tunable wavelength-selective element and a wavelength-selective element having a fixed transmission spectrum. By using a tunable wavelength selective element, as an example, differing wavelength ranges may be selected sequentially, whereas, by using a wavelength-selective element having a fixed transmission spectrum, the selection of the wavelength ranges may be fixed and may, however, be dependent e.g. on a detection position, thereby allowing, as an example, in the detection light beam path, for simultaneously exposing different detectors and/or different photosensitive detectors of the detector to differing spectral ranges of light.

Thus, as outlined above and as an example, the at least one filter element may comprise at least one of a filter, a grating, a prism, a plasmonic filter, a diffractive optical element and a metamaterial. More specifically, the spectrometer device may comprise at least one filter element disposed in a beam path of the light from the object, i.e. in the beam path of the detection light, wherein the filter element, specifically may be configured such that each of the photosensitive detectors is exposed to an individual spectral range of the light from the object. As an example, a variable filter element may be used, the transmission of which depends on a position on the filter element, such that, when the variable filter element is placed on top of the array of photosensitive detectors, the individual photosensitive detectors are exposed to differing spectral ranges of the incident light, specifically the detection light from the object. Additionally or alternatively the at least one wavelength-selective element may comprise at least one of the following elements: an array of individual bandpass filters, an array of patterned filters, an MEMS-interferometer, an MEMS-Fabry Perot interferometer. Further elements are feasible.

The spectrometer device may comprise at least one filter element disposed in a beam path of the light from the object, wherein the filter element may be configured such that the photosensitive detector may be exposed to the individual spectral range of the light from the object. The spectrometer device may comprise at least one further filter element disposed in a beam path of the light from the object, wherein the further filter element may be configured such that the photosensitive detector may be exposed to the further individual spectral range of the light from the object.

The spectrometer device comprises at least one evaluation unit, wherein the at least one evaluation unit is configured to consider the detector signals of the at least two photosensitive detectors for determining an item of reflectance information on the object.

The term "to evaluate", as used herein, is a broad term and is to be given its ordinary and customary meaning to a person of ordinary skill in the art and is not to be limited to a special or customized meaning. The term specifically may refer, without limitation, to the process of processing at least one first item of information in order to generate at least one second item of information thereby. Consequently, the term "evaluation unit", as used herein, is a broad term and is to be given its ordinary and customary meaning to a person of ordinary skill in the art and is not to be limited to a special or customized meaning. The term specifically may refer, without limitation, to an arbitrary device or a combination of devices configured to evaluate or process at least one first item of information, in order to generate at least one second item of information thereof. Thus, specifically, the evaluation unit may be configured for processing at least one input signal and to generate at least one output signal thereof. The at least one input signal, as an example, may comprise at least one detector signal provided directly or indirectly by the at least one photosensitive detector.

As an example, the evaluation unit may be or may comprise one or more integrated circuits, such as one or more application-specific integrated circuits (ASICs), and/or one or more data processing devices, such as one or more of computers, digital signal processors (DSP), field programmable gate arrays (FPGA) preferably one or more microcomputers and/or microcontrollers. Additional components may be comprised, such as one or more preprocessing devices and/or data acquisition devices, such as one or more devices for receiving and/or preprocessing of the detector signals, such as one or more AD-converters and/or one or more filters. Further, the evaluation unit may comprise one or more data storage devices. Further, the evaluation unit may comprise one or more interfaces, such as one or more wireless interfaces and/or one or more wire-bound interfaces.

The term "reflectance information", as used herein, is a broad term and is to be given its ordinary and customary meaning to a person of ordinary skill in the art and is not to be limited to a special or customized meaning. The term specifically may refer, without limitation, to an arbitrary information on a characteristic of the object related to the reflectivity of the object, specifically the effectiveness in reflecting light.

Considering the detector signals of the at least two photosensitive detectors for determining the item of reflectance information on the object may comprise comparing the detector signals of the at least two photosensitive detectors to one or more reference detector signals. The term "reference detector signals", as used herein, is a broad term and is to be given its ordinary and customary meaning to a person of ordinary skill in the art and is not to be limited to a special or customized meaning. The term specifically may refer, without limitation, to a standard signal used as a point of comparison for evaluating and/or measure at least one further value and/or quantity. The reference signal may be determined in a calibration procedure, particularly by determining the reference detector signal when using at least one standard object.

The one or more reference detector signals may be determined by using a calibration target having a known reflectance property, particularly a diffuse reflectance behavior, more particularly a diffuse reflectance behavior following the Lambert's cosine law. The term "Lambert's cosine law", as used herein, is a broad term and is to be given its ordinary and customary meaning to a person of ordinary skill in the art and is not to be limited to a special or customized meaning. The term specifically may refer, without limitation, to a law according to which the radiant intensity and/or luminous intensity observed from an ideal diffuse reflecting object is directly proportional to the cosine of an angle between the line of sight of an observer and a surface normal of the object.

The optical path of detection light from the object to the at least two photosensitive detectors may be identical to the optical path of detection light from the calibration target to the at least two photosensitive detectors. The term "optical path", as used herein, is a broad term and is to be given its ordinary and customary meaning to a person of ordinary skill in the art and is not to be limited to a special or customized meaning. The term specifically may refer, without limitation, to a trajectory of a light ray when propagating through an optical medium. For the optical path of detection light from the object to the at least two photosensitive detectors being identical to the optical path of detection light from the calibration target to the at least two photosensitive detectors, the spectrometer device used in the calibration procedure may have an identical arrangement, particularly of the components that influence the optical path, such as the sample interface, the light emitting element and/or the two photosensitive detectors. Alternatively or in addition, the calibration target may be arranged in the same pose, specifically the same position and/or the same orientation, relative to the sample interface as the object.

The term "pose" as used herein is a broad term and is to be given its ordinary and customary meaning to a person of ordinary skill in the art and is not to be limited to a special or customized meaning. The term specifically may refer, without limitation, to an orientation and/or a position of a rigid body. A pose may be described by using six components. For describing a position, three components may be used, such as coordinates associated with a center of mass of the rigid body in three-dimensional space. For describing an orientation, three components may be used, typically the Euler angles. Together, these components may describe the full pose of the rigid body.

Determining the item of reflectance information may comprise determining the reflectance $r(\lambda,\beta)$ of the object for a wavelength $\lambda$ and the angle of incidence $\beta$. The term "reflectance", as used herein, is a broad term and is to be given its ordinary and customary meaning to a person of ordinary skill in the art and is not to be limited to a special or customized meaning. The term specifically may refer, without limitation, to the effectiveness of an object in reflecting light depending on the wavelength $\lambda$ and the angle of incidence $\beta$. Reflectance may be a measure of the amount of light incident on the object that is reflected compared to the amount of light absorbed and/or transmitted by the object.

Particularly in a general case, the item of reflectance information may classify the object as an object having generic reflection properties when the object reflectance $r(\lambda,\beta)$ depends on the wavelength $\lambda$ and the angle of incidence $\beta$.

The term "generic reflection property", as used herein, is a broad term and is to be given its ordinary and customary meaning to a person of ordinary skill in the art and is not to be limited to a special or customized meaning. The term specifically may refer, without limitation, to a general reflection property that is exhibit by any object without having to object to show any specific and/or distinct behavior, particularly for classifying the object.

Determining the item of reflectance information on the object may comprise determining if the reflectance $r(\lambda,\beta)$ is wavelength dependent or independent. For determining if the reflectance $r(\lambda,\beta)$ is wavelength dependent or independent, at least one detector signal of a third photosensitive detector of the at least two photosensitive detectors may be considered, wherein the third photosensitive detector may be sensitive to detection light of a further spectral range that is different from the spectral range associated with the first photosensitive detector and associated with the second photosensitive detector, wherein an angle of incidence $\beta$ of detection light that is incident on the sample interface and propagating to the third photosensitive detector equals the angle of incidence $\beta$ of detection light that is incident on the sample interface and propagating to the first photosensitive detector or the angle of incidence $\beta$ of detection light that is incident on the sample interface and propagating to the second photosensitive detector.

Determining the reflectance $r(\lambda,\beta)$ of the object may comprise classifying the object into at least one type, particularly wherein the types may be selected from at least one of:

- a first type for an object having additive reflection properties;
- a second type for an object having multiplicative reflection properties;
- a third type for an object having diffuse reflection properties.

The object having additive reflection properties may be a first type specifying the generic reflection property. The object having multiplicative reflection properties may be a second type specifying the generic reflection property. The object having diffuse reflection properties may be a third type specifying the multiplicative reflection property.

The term "additive reflection properties", as used herein, is a broad term and is to be given its ordinary and customary meaning to a person of ordinary skill in the art and is not to be limited to a special or customized meaning. The term specifically may refer, without limitation, to reflection properties that may be described by the sum of an object surface associated contribution $S_{\mathrm{Surface}}(\beta)$ to the detector signal and an object volume associated contribution $S_{\mathrm{volume}}(\beta,\lambda)$ to the detector signal.

The item of reflectance information may classify the object as
an object of a first type, particularly having additive reflection properties, if the detector signal $S_\beta(\lambda)$ may be described by a sum of an object surface associated contribution $S_{\mathrm{Surface}}(\beta)$ to the detector signal and an object volume associated contribution $S_{\mathrm{volume}}(\beta,\lambda)$ to the detector signal.

Particularly in this case, the reflectance $r(\lambda,\beta)$ for a wavelength $\lambda$ and an angle of incidence $\beta$ may be given by equation (3)

$$r(\lambda,\beta) = \frac{S_\beta(\lambda)}{R_\beta(\lambda)} = \frac{S_{\mathrm{surface}}(\beta) + S_{\mathrm{volume}}(\beta,\lambda)}{R_\beta(\lambda)} = \frac{S_{\mathrm{surface}}(\beta) + f(\beta)\,S_0(\lambda)}{f(\beta) \cdot R_0(\lambda)}, \qquad (3)$$

wherein $S_\beta(\lambda)$ is the detector signal, $R_\beta(\lambda)$ is the reference signal, $S_{\mathrm{Surface}}(\beta)$ is an object surface associated contribution to the detector signal, $S_{\mathrm{volume}}(\beta,A)$ is an object volume associated contribution to the detector signal, $f(\beta)$ is the scattering function of the object (112) and of the calibration target, $S_0(\lambda)$ is the detector signal for $\beta=0$ and $R_0(\lambda)$ is the reference signal for $\beta=0$, particularly wherein $f(\beta)$ follows Lambert's cosine law and $f(\beta) = \cos(\beta)$.

For at least one angle of incidence $\beta$ that equals at least one angle of incidence $\alpha$ of the illumination light that is incident on the sample interface and propagating to the object

- an object surface associated contribution to the detector signal $S_{\mathrm{Surface}}(\beta)$, particularly depending on the angle of incidence $\beta$, and
- an object volume associated contribution to the detector signal $S_0(\lambda)$, specifically for $\beta = 0$, particularly depending on the wavelength $\lambda$,

may be determined, particularly by using a regression algorithm considering equation (3).

As used herein, the term "angle of incidence $\alpha$" is a broad term and is to be given its ordinary and customary meaning to a person of ordinary skill in the art and is not to be limited to a special or customized meaning. The term specifically may refer, without limitation, to an angle between the illumination light and the normal vector of the sample interface.

The term "multiplicative reflection properties", as used herein, is a broad term and is to be given its ordinary and customary meaning to a person of ordinary skill in the art and is not to be limited to a special or customized meaning. The term specifically may refer, without limitation, to reflection properties having a dependence on the angle of incidence $\beta$ that may be described by ratio of a scattering function $g(\beta)$ of the object and a different scattering function of the calibration target $f(\beta)$, particularly and a further wavelength dependent factor, specifically $r(\lambda, \beta = 0)$.

The item of reflectance information may classify the object as an object of a second type, particularly having multiplicative reflection properties, if the detector signal $S_\beta(\lambda)$ depends on the product of an angle independent function $S_0(\lambda)$ and a wavelength independent scattering function of the object $g(\beta)$, particularly wherein $g(\beta)$ is different from the scattering function of the calibration target $f(\beta)$.

Particularly in this case, the reflectance $r(\lambda, \beta)$ for a wavelength $\lambda$ and an angle of incidence $\beta$ may be given by equation (2)

$$r(\lambda, \beta) = \frac{S_\beta(\lambda)}{R_\beta(\lambda)} = \frac{g(\beta) \cdot S_0(\lambda)}{f(\beta) \cdot R_0(\lambda)} = \frac{\frac{g(\beta)}{f(\beta)} f(\beta) \cdot S_0(\lambda)}{f(\beta) \cdot R_0(\lambda)} = \frac{g(\beta)}{f(\beta)} \cdot r(\lambda, \beta = 0), \quad (2)$$

wherein $S_\beta(\lambda)$ is the detector signal, $R_\beta(\lambda)$ is the reference signal, $g(\beta)$ is the scattering function of the object (112), $f(\beta)$ is the scattering function of the calibration target, $S_0(\lambda)$ is the detector signal for $\beta = 0$ and $R_0(\lambda)$ is the reference signal for $\beta = 0$, particularly wherein $f(\beta)$ follows Lambert's cosine law $f(\beta)=\cos(\beta)$. The scattering function $g(\beta)$ may be determined, particularly by considering equation (2).

The term "diffuse reflection properties", as used herein, is a broad term and is to be given its ordinary and customary meaning to a person of ordinary skill in the art and is not to be limited to a special or customized meaning. The term specifically may refer, without limitation, to reflection properties that may be described by Lambert's cosine law.

The item of reflectance information may classify the object as an object of a third type, particularly having diffuse reflection properties, more particularly following the Lambert's cosine law, if the detector signal $S_\beta(\lambda)$ for a wavelength $\lambda$ is independent of an angle of incidence $\alpha$ of the illumination light that is incident on the sample interface and propagating to the object and is proportional to the cosine of the angle of incidence $\beta$.

Particularly in this case, the reflectance $r(\lambda, \beta)$ for a wavelength $\lambda$ and an angle of incidence $\beta$ may be given by equation (1)

$$r(\lambda, \beta) = \frac{S_\beta(\lambda)}{R_\beta(\lambda)} = \frac{f(\beta) \cdot S_0(\lambda)}{f(\beta) \cdot R_0(\lambda)} = \frac{S_0(\lambda)}{R_0(\lambda)}, \quad (1)$$

wherein $S_\beta(\lambda)$ is the detector signal, $R_\beta(\lambda)$ is the reference signal associated with the detector signal of a diffuse reference target, $f(\beta)$ is a scattering function of the object and the calibration target, $S_0(\lambda)$ is the detector signal for $\beta=0$ and $R_0(\lambda)$ is the reference signal for $\beta=0$, particularly wherein $f(\beta)=\cos(\beta)$.

The spectrometer device further may comprise a signal device, particularly a display, designated for indicating the item of reflectance information to a user. The signal device may typically be an arbitrary device giving at least one of: a haptic signal, an audible signal and/or a visual signal to a user. The signal device may be a display and/or a screen. Further, the signal device may be designated to indicate to a user to repeat the measurement when a classification of the object into a type failed.

The at least one evaluation unit may be further configured to determine at least one item of spectral information on the object. The at least one evaluation unit may be adapted to execute at least one computer program, such as at least one computer program performing or supporting the step of generating the items of information. As an example, one or more algorithms may be implemented which, by using the at least one detector signal, such as the time resolved detector signal and/or the spectroscopic detector signal, as input variables, may perform a predetermined transformation for deriving the spectroscopic information on the object, such as for deriving at least one spectroscopic information describing at least one property of the object. For this purpose, the evaluation unit may, particularly, comprise at least one data processing device, also referred to as a processor, in particular an electronic data processing device, which can be designed to generate the desired information by evaluating the detector signal. The evaluation unit may use an arbitrary process for generating the required information, such as by calculation and/or using at least one stored and/or known relationship. The evaluation unit specifically may be configured for performing at least one digital signal processing (DSP) technique on the primary detector signal or any secondary detector signal derived thereof, in particular at least one Fourier transformation.

Additionally or alternatively, the evaluation unit may be configured for performing one or more further digital signal processing techniques on the primary detector signal or any secondary detector signal derived thereof, e.g. windowing, filtering, Goertzel algorithm, crosscorrelation and autocorrelation. Besides the detector signal, one or a plurality of further parameters and/or items of information can influence said relationship. The relationship can be determined or determinable empirically, analytically or else semi-empirically. As an example, the relationship may comprise at least one of a model or calibration curve, at least one set of calibration curves, at least one function or a combination of the possibilities mentioned. One or a plurality of calibration curves can be stored for example in the form of a set of values and the associated function values thereof, for example in a data storage device and/or a table. Alternatively or additionally, however, the at least one calibration curve can also be stored for example in parameterized form and/or as a functional equation. Separate relationships for processing the detector signals into the items of information may be used. Alternatively, at least one combined relationship for processing the detector signals is feasible. Various possibilities are conceivable and can also be combined.

As an example, the detector signal may comprise a plurality of detector signals being at least a function of the wavelength of the detection light, and, optionally, also of time, specifically for time-dependent detector signals. This plurality of detector signals, such as the spectroscopic detector signal, may form a spectrum, including the option of a digital or an analogue spectrum. Thus, as an example, each of the detector signals may summarize information from a predetermined spectral range being defined by a spectral resolution of the detector. As will be outlined in further detail below, the detector may comprise exactly one or a plurality of photosensitive elements, each of the photosensitive elements being sensitive in a different spectral range and/or being exposed to a different part of the spectrum of the detection light. The entirety of the detector signals of the photosensitive elements may form the detector signal, or in the entirety, as an example, defines the spectral information, a part thereof, or a predecessor thereof. Since the spectral range of sensitivity of each of the photosensitive elements may be known, particularly from considering the time resolved detector signal, the intensity of the detection light as a function of the detection wavelength may be derived by this detector signal, by combining the data pairs of the photosensitive elements, each data pair comprising the respective signal of the photosensitive element and the wavelength of sensitivity. It shall be noted, however, that other ways of generating spectral information are also feasible, such as by sequentially exposing one and the same detector to different spectral portions of the detection light, e.g. by using a scannable wavelength-selective element.

As an example, the detector may be configured for generating a plurality of detector signals for at least one spectral range, specifically for at least two differing spectral ranges of the light from the object, specifically at least one of sequentially and simultaneously. For example, the detector, as outlined above, may comprise an array of photosensitive elements, wherein each photosensitive element may be sensitive in a different spectral range and/or may be exposed to light in a different spectral range. A plurality of detector signals sensitive to differing spectral ranges may be combined for generating the detector signal that is evaluated for deriving the spectroscopic information on the object.

For determining the item of spectral information on the object, at least one detector signal may be considered, particularly wherein the detector signal considered for determining the item of spectral information is associated with detection light. The detector signal may considered for determining at least one item of spectral information on the object is generated by at least of:

- at least one photosensitive detector of the two photosensitive detectors considered for determining the item of reflectance information on the object;
- at least one further photosensitive detector.

The at least one evaluation unit may be further configured to provide the spectral information on the object when the item of reflectance information indicates a reflectance type of the object.

In a further aspect, a method for determining reflectance information on the object is disclosed. The method comprises using a spectrometer device as discussed elsewhere herein, wherein a reflectance information on the object is determined by considering the detector signals of the at least two photosensitive detectors. The method according to the preceding claim, wherein the method is computer-implemented.

In a further aspect, a computer program is disclosed, comprising instructions which, when the program is executed by the spectrometer device according to any one of the preceding claims referring to a spectrometer device cause the evaluation unit of the spectrometer device to determine at least one item of reflectance information about the at least one measurement object by considering a detector signal. Specifically, the computer program may be stored on a computer-readable data carrier and/or on a computer-readable storage medium. The computer program may be executed on at least one processor comprised by the optoelectronic apparatus and/or the device.

In a further aspect, a computer-readable storage medium is disclosed, comprising instructions which, when the program is executed by the spectrometer device according to any one of the preceding claims referring to a spectrometer device cause the evaluation unit of the spectrometer device to determine at least one item of reflectance information about the at least one measurement object by considering a detector signal.

In a further aspect, a non-transient computer-readable medium is disclosed, including instructions that, when executed by one or more processors of an evaluation unit of a spectrometer device according to any one of the preceding claims referring to a spectrometer device cause one or more processors to determine at least one item of reflectance information about the at least one measurement object by considering a detector signal.

As used herein, the terms "computer-readable data carrier" and "computer-readable storage medium" specifically may refer to non-transitory data storage means, such as a hardware storage medium having stored thereon computer-executable instructions. The stored computer-executable instruction may be associate with the computer program. The computer-readable data carrier or storage medium specifically may be or may comprise a storage medium such as a random-access memory (RAM) and/or a read-only memory (ROM).

As used herein, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically are used only once when introducing the respective feature or element. In most cases, when referring to the respective feature or element, the expressions "at least one" or "one or more" are not repeated, non withstanding the fact that the respective feature or element may be present once or more than once.

Further, as used herein, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The present disclosure exhibits several advantages, discussed in the following. The spectrometer device is capable of determining and/or accounting for the scattering probabilities of a sample. This can enable differentiation not only of different materials, but also fabrication techniques.

Overall, in the context of the present invention, the following embodiments are regarded as preferred:

Embodiment 1. A spectrometer device for determining an item of spectral information on at least one object, the spectrometer device comprising:

- at least one sample interface, wherein the sample interface is designated to define a measurement pose of the object;
- at least one light emitting element, wherein the at least one light emitting element is designated to emit illumination light onto the object for generating detection light;
- at least two photosensitive detectors designated to generate at least one detector signal when receiving detection light, wherein a first angle of incidence $\beta$ of detection light that is incident on the sample interface and propagating to a first photosensitive detector of the at least two photosensitive detectors is different from a second angle of incidence $\beta$ of detection light that is incident on the sample interface and propagating to a second photosensitive detector of the at least two photosensitive detectors, wherein the first photosensitive detector and the second photosensitive detector detect light of the same spectral range of the detection light;
- at least one evaluation unit, wherein the at least one evaluation unit is configured to consider the detector signals of the at least two photosensitive detectors for determining an item of reflectance information on the object.

Embodiment 2. The spectrometer device according to the preceding Embodiment, wherein considering the detector signals of the at least two photosensitive detectors for determining the item of reflectance information on the object comprises comparing the detector signals of the at least two photosensitive detectors to one or more reference detector signals.

Embodiment 3. The spectrometer device according to the preceding Embodiment, wherein the one or more reference detector signals are determined by using a calibration target having a known reflectance property, particularly a diffuse reflectance behavior, more particularly a diffuse reflectance behavior following the Lambert's cosine law.

Embodiment 4. The spectrometer device according to any one of the two preceding Embodiments, wherein the optical path of detection light from the object to the at least two photosensitive detectors is identical to the optical path of detection light from the calibration target to the at least two photosensitive detectors.

Embodiment 5. The spectrometer device according to any one of the preceding Embodiments, wherein determining the item of reflectance information comprises determining the reflectance $r(\lambda,\beta)$ of the object for a wavelength $\lambda$ and the angle of incidence $\beta$.

Embodiment 6. The spectrometer device according to any one of the preceding Embodiments, wherein, particularly in a general case, the item of reflectance information classifies the object as
an object having generic reflection properties when the object reflectance $r(\lambda,\beta)$ depends on the wavelength $\lambda$ and the angle of incidence $\beta$.

Embodiment 7. The spectrometer device according to any one of the preceding Embodiments, wherein determining the item of reflectance information on the object comprises determining if the reflectance $r(\lambda,\beta)$ is wavelength dependent or independent.

Embodiment 8. The spectrometer device according to the preceding Embodiment,

wherein, for determining if the reflectance $r(\lambda,\beta)$ is wavelength dependent or independent, at least one detector signal of a third photosensitive detector of the at least two photosensitive detectors is considered, wherein the third photosensitive detector is sensitive to detection light of a further spectral range that is different from the spectral range associated with the first photosensitive detector and associated with the second photosensitive detector, wherein an angle of incidence $\beta$ of detection light that is incident on the sample interface and propagating to the third photosensitive detector equals the angle of incidence $\beta$ of detection light that is incident on the sample interface and
propagating to the first photosensitive detector or the angle of incidence $\beta$ of detection light that is incident on the sample interface and propagating to the second photosensitive detector.

Embodiment 9. The spectrometer device according to any one of the four preceding Embodiments, wherein determining the reflectance $r(\lambda,\beta)$ of the object comprises classifying the object into at least one type, wherein the types is selected from at least one of:

- a first type for an object having additive reflection properties;
- a second type for an object having multiplicative reflection properties;
- a third type for an object having diffuse reflection properties.

Embodiment 10. The spectrometer device according to any one of the preceding Embodiments, wherein the item of reflectance information classifies the object as an object of a first type, particularly having additive reflection properties, if the detector signal $S_\beta(\lambda)$ depends on a sum of an object surface associated contribution $S_{\text{Surface}}(\beta)$ to the detector signal and an object volume associated contribution $S_{\text{volume}}(\beta,A)$ to the detector signal.

Embodiment 11. The spectrometer device according to the preceding Embodiment,
wherein the reflectance $r(\lambda,\beta)$ for a wavelength $\lambda$ and an angle of incidence $\beta$ is given by equation (3)

$$r(\lambda,\beta) = \frac{S_\beta(\lambda)}{R_\beta(\lambda)} = \frac{S_{\text{surface}}(\beta)+S_{\text{volume}}(\beta,\lambda)}{R_\beta(\lambda)} = \frac{S_{\text{surface}}(\beta)+f(\beta)\,S_0(\lambda)}{f(\beta)\cdot R_0(\lambda)}, \qquad (3)$$

wherein $S_\beta(\lambda)$ is the detector signal, $R_\beta(\lambda)$ is the reference signal, $S_{\text{Surface}}(\beta)$ is an object surface associated contribution to the detector signal, $S_{\text{volume}}(\beta,A)$ is an object volume associated contribution to the detector signal, $f(\beta)$ is the scattering function of the object and of the calibration target, $S_0(\lambda)$ is the detector signal for $\beta=0$ and $R_0(\lambda)$ is the reference signal for $\beta=0$, particularly wherein $f(\beta)$ follows Lambert's cosine law and $f(\beta) = \cos(\beta)$.

Embodiment 12. The spectrometer device according to the two preceding Embodiments, for at least one angle of incidence $\beta$ that equals at least one angle of incidence $\alpha$ of the illumination light that is incident on the sample interface and propagating to the object

- an object surface associated contribution to the detector signal $S_{\text{Surface}}(\beta)$, particularly depending on the angle of incidence $\beta$, and
- an object volume associated contribution to the detector signal $S_0(\lambda)$, specifically for $\beta=0$, particularly depending on the wavelength $\lambda$,

are determined, particularly by using a regression algorithm considering equation (3).

Embodiment 13. The spectrometer device according to any one of the preceding Embodiments, wherein the item of reflectance information classifies the object as
an object of a second type, particularly having multiplicative reflection properties, if the detector signal $S_\beta(\lambda)$ depends on the product of an angle independent function $S_0(\lambda)$ and a wavelength independent scattering function of the object $g(\beta)$, particularly wherein $g(\beta)$ is different from the scattering function of the calibration target $f(\beta)$.

Embodiment 14. The spectrometer device according to the preceding Embodiment,
wherein the reflectance $r(\lambda,\beta)$ for a wavelength $\lambda$ and an angle of incidence $\beta$ is given by equation (2)

$$r(\lambda,\beta) = \frac{S_\beta(\lambda)}{R_\beta(\lambda)} = \frac{g(\beta)\cdot S_0(\lambda)}{f(\beta)\cdot R_0(\lambda)} = \frac{\frac{g(\beta)}{f(\beta)}f(\beta)\cdot S_0(\lambda)}{f(\beta)\cdot R_0(\lambda)} = \frac{g(\beta)}{f(\beta)}\cdot r(\lambda,\beta=0),\ (2)$$

wherein $S_\beta(\lambda)$ is the detector signal, $R_\beta(\lambda)$ is the reference signal, $g(\beta)$ is the scattering function of the object (112), $f(\beta)$ is the scattering function of the calibration target, $S_0(\lambda)$ is the detector signal for $\beta=0$ and $R_0(\lambda)$ is the reference signal for $\beta=0$, particularly wherein $f(\beta)$ follows Lambert's cosine law $f(\beta)=\cos(\beta)$.

Embodiment 15. The spectrometer device according to the preceding Embodiment,
wherein the scattering function $g(\beta)$ is determined, particularly by considering equation (2).

Embodiment 16. The spectrometer device according to any one of the preceding Embodiments, wherein the item of reflectance information classifies the object as an object of a third type, particularly having diffuse reflection properties, more particularly following the Lambert's cosine law, if the detector signal $S_\beta(\lambda)$ for a wavelength $\lambda$ is independent of an angle of incidence $\alpha$ of the illumination light that is incident on the sample interface and propagating to the object and is proportional to the cosine of the angle of incidence $\beta$.

Embodiment 17. The spectrometer device according to the preceding Embodiment,
wherein the reflectance $r(\lambda,\beta)$ for a wavelength $\lambda$ and an angle of incidence $\beta$ is given by equation (1)

$$r(\lambda,\beta) = \frac{S_\beta(\lambda)}{R_\beta(\lambda)} = \frac{f(\beta)\cdot S_0(\lambda)}{f(\beta)\cdot R_0(\lambda)} = \frac{S_0(\lambda)}{R_0(\lambda)},\qquad (1)$$

wherein $S_\beta(\lambda)$ is the detector signal, $R_\beta(\lambda)$ is the reference signal associated with the detector signal of a diffuse reference target, $f(\beta)$ is a scattering function of the object and the calibration target, $S_0(\lambda)$ is the detector signal for $\beta=0$ and $R_0(\lambda)$ is the reference signal for $\beta=0$, particularly wherein $f(\beta) = \cos(\beta)$.

Embodiment 18. The spectrometer device according to any one of the preceding Embodiments, wherein the sample interface is a measurement window, particularly comprising silicone, designated for transmitting the illumination light and the detection light.

Embodiment 19. The spectrometer device according to any one of the preceding Embodiments, wherein the light emitting element is at least one of:

- a thermal radiator;
- a laser, specifically a vertical cavity surface emitting laser (VCSEL), particularly emitting at least one wavelength in the infrared region;
- a light-emitting diode (LED), particularly

  ◦ a LED emitting light that is at least partially located in the infrared spectral range and/or
  ◦ a LED illuminating a phosphor for light-conversion of light generated by the LED, wherein the luminescent material generates converted light that is at least partly located in the near-infrared spectral range.

Embodiment 20. The spectrometer device according to the preceding Embodiment, wherein the thermal radiator is selected from an incandescent lamp or a thermal infrared emitter.

Embodiment 21. The spectrometer device according to any one of the preceding Embodiments, wherein the at least two photosensitive detectors are designated to receive detection light reflected and/or scattered by the object.

Embodiment 22. The spectrometer device according to any one of the preceding Embodiments, wherein the spectrometer device further comprises a signal device, particularly a display, designated for indicating the item of reflectance information to a user.

Embodiment 23. The spectrometer device according to the preceding Embodiment, wherein the signal device is further designated to indicate to a user to repeat the measurement when a classification of the object into a type failed.

Embodiment 24. The spectrometer device according to any one of the preceding Embodiments, wherein the at least one evaluation unit is further configured to determine at least one item of spectral information on the object.

Embodiment 25. The spectrometer device according to the preceding Embodiment, wherein, for determining the item of spectral information on the object, at least one detector signal is considered, particularly wherein the detector signal considered for determining the item of spectral information is associated with detection light.

Embodiment 26. The spectrometer device according to the preceding Embodiment, wherein the detector signal considered for determining at least one item of spectral information on the object is generated by at least of:

- at least one photosensitive detector of the two photosensitive detectors considered for determining the item of reflectance information on the object;
- at least one further photosensitive detector.

Embodiment 27. The spectrometer device according to any one of the three preceding Embodiments, wherein the at least one evaluation unit is further configured to provide the spectral information on the object when the item of reflectance information indicates a reflectance type of the object.

Embodiment 28. A method for determining reflectance information on the object, wherein the method comprising using a spectrometer device according to any one of the preceding Embodiments, wherein a reflectance information on the object is determined by considering the detector signals of the at least two photosensitive detectors.

Embodiment 29. The method according to the preceding Embodiment, wherein the method is computer-implemented.

Embodiment 30. A computer program comprising instructions which, when the program is executed by the spectrometer device according to any one of the preceding Embodiments referring to a spectrometer device cause the evaluation unit of the spectrometer device to determine at least one item of reflectance information about the at least one measurement object by considering a detector signal.

Embodiment 31. A computer-readable storage medium comprising instructions which, when the program is executed by the spectrometer device according to any one of the preceding Embodiments referring to a spectrometer device cause the evaluation unit of the spectrometer device to determine at least one item of reflectance information about the at least one measurement object by considering a detector signal.

Embodiment 32. A non-transient computer-readable medium including instructions that, when executed by one or more processors of an evaluation unit of a spectrometer device according to any one of the preceding Embodiments referring to a spectrometer device cause one or more processors to determine at least one item of reflectance information about the at least one measurement object by considering a detector signal.

Brief description of the figures

Further optional details and features of the invention are evident from the description of preferred exemplary embodiments which follows in conjunction with the dependent claims. In this context, the particular features may be implemented in an isolated fashion or in combination with other features. The invention is not restricted to the exemplary embodiments. The exemplary embodiments are shown schematically in the figures. Identical reference numerals in the individual figures refer to identical elements or elements with identical function, or elements which correspond to one another with regard to their functions.

Specifically, in the figures:

Figure 1    shows a dependency of a detector signal on an angle of incidence β;

Figure 2    shows an exemplary spectrometer device; and

Figure 3    shows an exemplary arrangement of photosensitive detectors.

Detailed description of the embodiments:

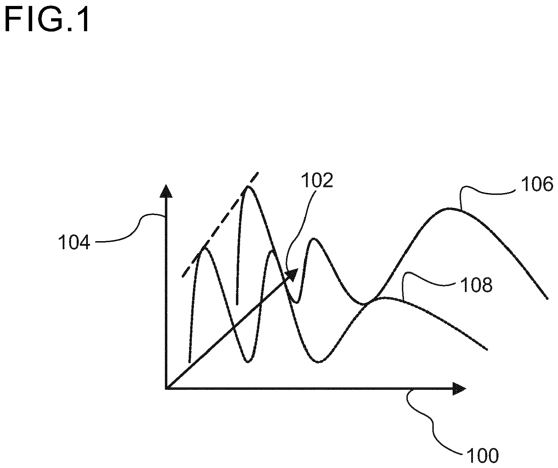

Figure 1 illustrates exemplarily the dependency of a signal level 106, 108 on the wavelength λ and the angle of incidence β that is incident on a sample interface 130 and propagating onto a photosensitive detector 122, 123. On the first horizontal axis 100, the wavelength λ is depicted. On the second horizontal axis 102, the angle of incidence β is depicted. On the vertical axis 104, the strength of signal level is depicted. The dashed line illustrates that the strength of the signal levels 106, 108 for a given wavelength λ is different for different angles of incidence β. The present invention takes advantage of this effect.

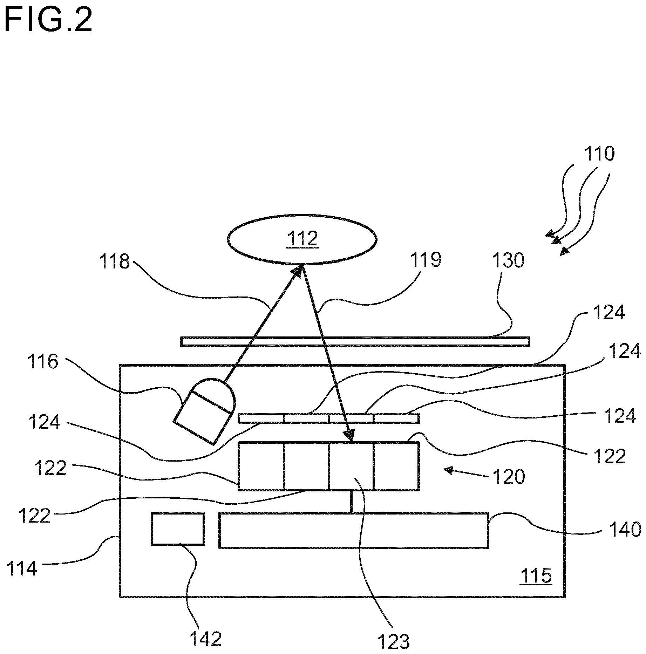

Figure 2 illustrates, in a highly schematic fashion, an exemplary spectrometer device 110 for determining an item of spectral information on at least one object 112 according to the present invention. The object 112 may be or comprise an arbitrary body, chosen from a living object and a non-living object, which comprises material for investigation or monitoring by the spectrometer device 110.

According to the present invention, the spectrometer device 110 may be adapted for recording a spectrum, particularly in the infrared (IR) spectral region, especially in the near-infrared (NIR), specifically for a wavelength of 760 nm to 3 μm, preferably of 1 μm to 5 μm, more preferred of 1 μm to 3 μm, or in the mid-infrared spectral region which covers wavelengths from 5 μm to 15 μm. Accordingly, the spectrometer device 110 can be used for monitoring or investigation purposes, such as spectroscopy, gas sensing, or concentration measurements. However, further applications of the spectrometer device 110 may also be feasible.

As depicted in Figure 2, the spectrometer device 110 may comprise a housing 114, which houses the components of the spectrometer device 110. In this manner, the components of the spectrometer device 110 can be protected and access of external light may be impeded. Further, the housing 114 can, preferably, be filled with an optically transparent medium 115, wherein the optically transparent medium 115 may, especially, be selected from ambient air, an inert gas or vacuum, especially in order to facilitate a propagation of the light beam within the housing 114. However, different kind of materials may also be feasible. Further kinds of arrangements of the components of the spectrometer device 110 may also be conceivable.

The exemplary spectrometer device 110 as schematically depicted in Figure 2 may comprise a light emitting element 116 which is designated to emit illumination light 118 onto the object 112 for generating detection light 119. The light emitting element 116 may be a thermal radiator. The thermal radiator may be selected from an incandescent lamp and/or a thermal infrared emitter. Alternatively or in addition, the light emitting element 116 may be a laser, specifically a vertical cavity surface emitting laser (VCSEL), particularly emitting at least one wavelength in the infrared region. Alternatively or in addition, the light emitting element 116 may be a light-emitting diode (LED), particularly a LED emitting light that is at least partially located in the infrared spectral range and/or a LED illuminating a phosphor for light-conversion of light generated by the LED, wherein the luminescent material generates converted light that is at least partly located in the near-infrared spectral range. The light emitting element 116 may be continuously emitting, or generating modulated optical pulses. The light emitting element 116 may be configured for emitting the light 118 isotropically in all spatial directions. However, the light emitting element 116 may, preferably, be designed for emitting the light 118 anisotropically in at least one spatial direction, specifically towards the at least one measurement object 112, such as by generating at least one light beam.

The exemplary spectrometer device 110 as illustrated in Figure 2, further, comprises at least one sample interface 130, wherein the sample interface 130 is designated to define a measurement pose of the object 112. The sample interface 130 may be a measurement window, particularly comprising silicone, designated for transmitting the illumination light 118 and/or the detection light 119. The at least one sample interface 130 may be at least partially, preferably fully, transparent for the respective light 118, 119. The sample interface 130, particularly when being a measurement window and defining an aperture, may, especially, be located in a portion of the housing 114 of the spectrometer device 110, specifically in a portion of a beam path of illumination light 118 and/or the detection light 119.

Further, the exemplary spectrometer device 110 as schematically illustrated in Figure 2 comprises at least two photosensitive detectors 122, particularly comprised by a detector array 120, that generate at least one detector signal when receiving light, specifically detection light 119, wherein the photosensitive detectors 122 are designated to receive at least a portion of the generated detection light 119.

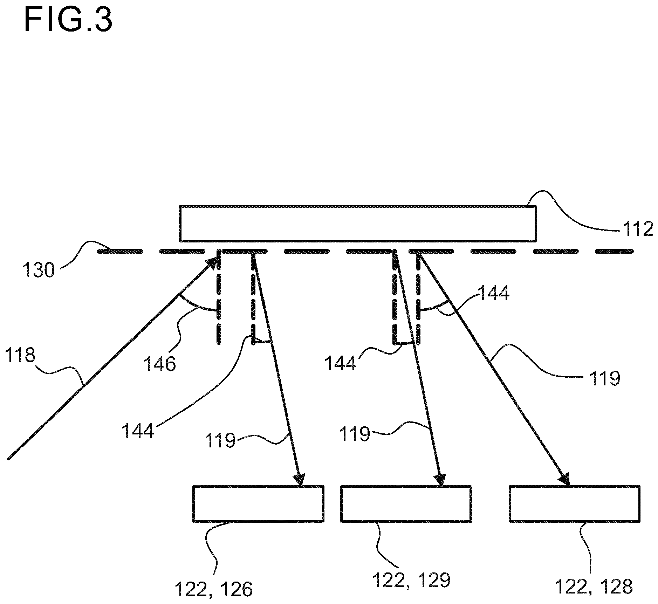

In Figure 3, a schematic view of an exemplary arrangement of the at least two photosensitive detectors 122 is illustrated. The illumination light 118 is incident onto the object 112 at an angle of incidence $\alpha$ of illumination light that is incident on the sample interface 130 and propagating to the object 146. The angle of incidence $\alpha$ may be defined by the angle between the illumination light 118 and the normal vector (illustrated by the vertical dashed lines in Figure 3) of the sample interface 130. A first angle $\beta$ of incidence of detection light 144 that is incident on the sample interface and propagating to a first photosensitive detector 126 of the at least two photosensitive detectors 122, is different from a second angle $\beta$ of incidence of detection light 144 that is incident on the sample interface and propagating to a second photosensitive detector 128 of the at least two photosensitive detectors 122. The first photosensitive detector 126 and the second photosensitive detector 128 detect light of the same spectral range of the detection light 119.

For detecting light of the same spectral range of the detection light 119, at least one filter element 124 may be arranged in a beam path of the detection light 119, as exemplarily illustrated in Figure 2. The filter element 124 may be configured such that the first photosensitive detector 126 and the second photosensitive detector 128 are exposed to detection light 119 of the same spectral range. The filter element 124 may be selected from the group of a tunable wavelength-selective element and a wavelength-selective element having a fixed transmission spectrum. The at least two photosensitive detectors may be designated to receive detection light 119 reflected and/or scattered by the object 112.

Further, the exemplary spectrometer device 110 as schematically illustrated in Figure 2 comprises at least one evaluation unit 140, wherein the at least one evaluation unit 140 is configured to consider the detector signals of the at least two photosensitive detectors 122 for determining an item of reflectance information on the object 112. Determining the item of reflectance information may comprise determining the reflectance $r(\lambda,\beta)$ of the object 112 for a wavelength $\lambda$ and the angle of incidence $\beta$.

Considering the detector signals of the at least two photosensitive detectors 122 for determining the item of reflectance information on the object 112 may comprise comparing the detector signals of the at least two photosensitive detectors 122 to one or more reference detector signals. The one or more reference detector signals may be determined by using a calibration target having a known reflectance property, particularly a diffuse reflectance behavior, more particularly a diffuse reflectance behavior following the Lambert's cosine law. The optical path of detection light 119 from the object 112 to the at least two photosensitive detectors 122 may be identical to the optical path of detection light 119 from the calibration target to the at least two photosensitive detectors 122.

Determining the item of reflectance information on the object 112 may comprise determining if the reflectance $r(\lambda,\beta)$ is wavelength dependent or independent, particularly by further considering at least one detector signal of a third photosensitive detector 129, as exemplarily illustrated in Figures 3, of the at least two photosensitive detectors 122. The third photosensitive detector 129 may be sensitive to detection light 119 of a further spectral range that is different from the spectral range associated with the first photosensitive detector 126 and associated with the second photosensitive detector 128. An angle of incidence $\beta$ of detection light 119 that is incident on the sample interface and propagating to the third photosensitive detector 129 may equal the angle of incidence $\beta$ of detection light 119 that is incident on the sample interface and propagating to the first photosensitive detector 126 or the angle of incidence $\beta$ of detection light 119 that is incident on the sample interface and propagating to the second photosensitive detector 128.

In a general case, the item of reflectance information may classify the object (112) as an object 112 having generic reflection properties when the object reflectance $r(\lambda,\beta)$ depends on the wavelength $\lambda$ and the angle of incidence $\beta$.

Determining the reflectance $r(\lambda,\beta)$ of the object 112 may comprise classifying the object 112 into at least one type, wherein the types is selected from at least one of:

- a first type for an object 112 having additive reflection properties;
- a second type for an object 112 having multiplicative reflection properties;
- a third type for an object 112 having diffuse reflection properties.

The item of reflectance information may comprise information, and particularly then classify the object 112 as an object 112 of a first type, particularly having additive reflection properties, if the detector signal $S_\beta(\lambda)$ depends on a sum of an object surface associated contribution $S_{\text{Surface}}(\beta)$ to the detector signal and an object volume associated contribution $S_{\text{volume}}(\beta,\lambda)$ to the detector signal.

Particularly in this case, the reflectance $r(\lambda,\beta)$ for a wavelength $\lambda$ and an angle of incidence $\beta$ may be given by equation (3)

$$r(\lambda,\beta) = \frac{S_\beta(\lambda)}{R_\beta(\lambda)} = \frac{S_{\text{surface}}(\beta) + S_{\text{volume}}(\beta,\lambda)}{R_\beta(\lambda)} = \frac{S_{\text{surface}}(\beta) + f(\beta)\, S_0(\lambda)}{f(\beta) \cdot R_0(\lambda)}, \qquad (3)$$

wherein $S_\beta(\lambda)$ is the detector signal, $R_\beta(\lambda)$ is the reference signal, $S_{\text{Surface}}(\beta)$ is an object surface associated contribution to the detector signal, $S_{\text{volume}}(\beta,\lambda)$ is an object volume associated contribution to the detector signal, $f(\beta)$ is the scattering function of the object 112 and of the calibration target, $S_0(\lambda)$ is the detector signal for $\beta=0$ and $R_0(\lambda)$ is the reference signal for $\beta=0$, particularly wherein $f(\beta)$ follows Lambert's cosine law and $f(\beta) = \cos(\beta)$.

Further particularly in this case, at least one angle of incidence $\beta$ that equals at least one angle of incidence $\alpha$ of the illumination light 118 that is incident on the sample interface 130 and propagating to the object 112

- an object surface associated contribution to the detector signal $S_{\text{Surface}}(\beta)$, particularly depending on the angle of incidence $\beta$, and
- an object volume associated contribution to the detector signal $S_0(\lambda)$, specifically for $\beta = 0$, particularly depending on the wavelength $\lambda$,

may be determined, particularly by using a regression algorithm considering equation (3).

The item of reflectance information may comprise information, and particularly then classify the object 112 as an object 112 of a second type, particularly having multiplicative reflection properties, if the detector signal $S_\beta(\lambda)$ depends on the product of an angle independent function $S_0(\lambda)$ and a wavelength independent scattering function of the object $g(\beta)$, particularly wherein $g(\beta)$ is different from the scattering function of the calibration target $f(\beta)$. Particularly in this case, the reflectance $r(\lambda,\beta)$ for a wavelength $\lambda$ and an angle of incidence $\beta$ is given by equation (2)

$$r(\lambda,\beta) = \frac{S_\beta(\lambda)}{R_\beta(\lambda)} = \frac{g(\beta)\cdot S_0(\lambda)}{f(\beta)\cdot R_0(\lambda)} = \frac{\frac{g(\beta)}{f(\beta)}f(\beta)\cdot S_0(\lambda)}{f(\beta)\cdot R_0(\lambda)} = \frac{g(\beta)}{f(\beta)} \cdot r(\lambda,\beta = 0), \quad (2)$$

wherein $S_\beta(\lambda)$ is the detector signal, $R_\beta(\lambda)$ is the reference signal, $g(\beta)$ is the scattering function of the object 112, $f(\beta)$ is the scattering function of the calibration target, $S_0(\lambda)$ is the detector signal for $\beta = 0$ and $R_0(\lambda)$ is the reference signal for $\beta=0$, particularly wherein $f(\beta)$ follows Lambert's cosine law $f(\beta) = \cos(\beta)$. The scattering function $g(\beta)$ may be determined, particularly by considering equation (2).

The item of reflectance information may comprise information, and particularly then classify the object 112 as an object 112 of a third type, particularly having diffuse reflection properties, more particularly following the Lambert's cosine law, if the detector signal $S_\beta(\lambda)$ for a wavelength $\lambda$ is independent of an angle of incidence $\alpha$ of the illumination light 118 that is incident on the sample interface and propagating to the object 112 and is proportional to the cosine of the angle of incidence $\beta$. Particularly in this case, the reflectance $r(\lambda,\beta)$ for a wavelength $\lambda$ and an angle of incidence $\beta$ may given by equation (1)

$$r(\lambda,\beta) = \frac{S_\beta(\lambda)}{R_\beta(\lambda)} = \frac{f(\beta)\cdot S_0(\lambda)}{f(\beta)\cdot R_0(\lambda)} = \frac{S_0(\lambda)}{R_0(\lambda)}, \quad (1)$$

wherein $S_\beta(\lambda)$ is the detector signal, $R_\beta(\lambda)$ is the reference signal associated with the detector signal of a diffuse reference target, $f(\beta)$ is a scattering function of the object 112 and the calibration target, $S_0(\lambda)$ is the detector signal for $\beta = 0$ and $R_0(\lambda)$ is the reference signal for $\beta= 0$, particularly wherein $f(\beta) = \cos(\beta)$.

The at least one evaluation unit 140 may be further configured to determine at least one item of spectral information on the object 112. For determining the item of spectral information on the object 112, at least one detector signal may be considered, particularly wherein the detector signal considered for determining the item of spectral information may be associated with detection light 119.

The detector signal considered for determining at least one item of spectral information on the object 112 may be generated by at least of: at least one photosensitive detector 126, 128, 129 of the two photosensitive detectors 122 considered for determining the item of reflectance information on the object 112; at least one further photosensitive detector 123. The at least one further photosensitive detector 123 may not be considered when deriving the item of reflectance information and/or the item of spectral information. The at least one further photosensitive detector 123 may be sensitive to at least one wavelength range differing from the wavelength range of at least one detector 126, 128, 129 of the two photosensitive detectors 122 may be sensitive to.

The spectrometer device 110 may further comprise a signal device, particularly a display 142, designated for indicating the item of reflectance information to a user. The signal device may be further designated to indicate to a user to repeat the measurement when a classification of the object 112 into a type failed. In such a case, the user may be indicated to repeat the measurement. The at least one evaluation unit 140 may further be configured to provide the spectral information on the object 112 when the item of reflectance information indicates a reflectance type of the object 112, particularly by displaying the item of reflectance information.

Further a method for determining reflectance information on the object 112, wherein the method comprising using a spectrometer device 110 as described elsewhere herein, wherein a reflectance information on the object 112 is determined by considering the detector signals of the at least two photosensitive detectors 122. The method may be computer-implemented. Further a computer program is disclosed comprising instructions which, when the program is executed by the spectrometer device 110 as described elsewhere herein cause the evaluation unit 140 of the spectrometer device 110 to determine at least one item of reflectance information about the at least one measurement object 112 by considering a detector signal. Further a computer-readable storage medium is disclosed comprising instructions which, when the program is executed by the spectrometer device 110 as described elsewhere herein cause the evaluation unit 140 of the spectrometer device 110 to determine at least one item of reflectance information about the at least one measurement object 112 by considering a detector signal. Further a non-transient computer-readable medium is disclosed including instructions that, when executed by one or more processors of an evaluation unit 140 of a spectrometer device 110 as described elsewhere herein cause one or more processors to determine at least one item of reflectance information about the at least one measurement object 112 by considering a detector signal.

List of reference numbers

[0109]

| | |
|---|---|
| 100 | first horizontal axis |
| 102 | second horizontal axis |
| 104 | vertical axis |
| 106 | signal level |
| 108 | signal level |
| 110 | spectrometer device |
| 112 | object |
| 114 | housing |
| 115 | optically transparent medium |
| 116 | light emitting element |
| 118 | illumination light |
| 119 | detection light |
| 120 | detector array |
| 122 | photosensitive detectors considered for deriving item of reflectance information |
| 123 | further photosensitive detector |
| 124 | filter element |
| 126 | first photosensitive detector |
| 128 | second photosensitive detector |
| 129 | third photosensitive detector |
| 130 | sample interface |
| 140 | evaluation unit |
| 142 | display |
| 144 | angle of incidence β |
| 146 | angle of incidence α |

**Claims**

1.  A spectrometer device (110) for determining an item of spectral information on at least one object (112), the spectrometer device (110) comprising:

    - at least one sample interface (130), wherein the sample interface (130) is designated to define a measurement pose of the object (112);
    - at least one light emitting element (116), wherein the at least one light emitting element (116) is designated to emit illumination light (118) onto the object (112) for generating detection light (119);
    - at least two photosensitive detectors (122) designated to generate at least one detector signal when receiving detection light (119), wherein a first angle of incidence $\beta$ of detection light (119) that is incident on the sample interface (130) and propagating to a first photosensitive detector (126) of the at least two photosensitive detectors (122) is different from a second angle of incidence $\beta$ of detection light (119) that is incident on the sample interface (130) and propagating to a second photosensitive detector (128) of the at least two photosensitive detectors (122), wherein the first photosensitive detector (126) and the second photosensitive detector (128) detect light of the same spectral range of the detection light (119);

- at least one evaluation unit (140), wherein the at least one evaluation unit (140) is configured to consider the detector signals of the at least two photosensitive detectors (122) for determining an item of reflectance information on the object (112).

2. The spectrometer device (110) according to the preceding claim, wherein considering the detector signals of the at least two photosensitive detectors (122) for determining the item of reflectance information on the object (112) comprises comparing the detector signals of the at least two photosensitive detectors (122) to one or more reference detector signals.

3. The spectrometer device (110) according to any one of the preceding claims, wherein determining the item of reflectance information comprises determining the reflectance $r(\lambda,\beta)$ of the object (112) for a wavelength $\lambda$ and the angle of incidence $\beta$.

4. The spectrometer device (110) according to the preceding claim, wherein determining the reflectance $r(\lambda,\beta)$ comprises determining if the reflectance $r(\lambda,\beta)$ is wavelength dependent or independent, wherein, for determining if the reflectance $r(\lambda,\beta)$ is wavelength dependent or independent, at least one detector signal of a third photosensitive detector (129) of the at least two photosensitive detectors (122) is considered, wherein the third photosensitive detector (129) is sensitive to detection light (119) of a further spectral range that is different from the spectral range associated with the first photosensitive detector (126) and associated with the second photosensitive detector (128), wherein an angle of incidence $\beta$ of detection light (119) that is incident on the sample interface (130) and propagating to the third photosensitive detector (129) equals the angle of incidence $\beta$ of detection light (119) that is incident on the sample interface (130) and propagating to the first photosensitive detector (126) or the angle of incidence $\beta$ of detection light (119) that is incident on the sample interface (130) and propagating to the second photosensitive detector (128).

5. The spectrometer device (110) according to the preceding claim, wherein the item of reflectance information classifies the object (112) as
an object (112) of a first type, particularly having additive reflection properties, when the detector signal $S_\beta(\lambda)$ depends on a sum of an object surface associated contribution $S_{Surface}(\beta)$ to the detector signal and an object volume associated contribution $S_{volume}(\beta,A)$ to the detector signal.

6. The spectrometer device (110) according to any one of the preceding claims, wherein the item of reflectance information classifies the object (112) as
an object (112) of a second type, particularly having multiplicative reflection properties, when the detector signal $S_\beta(\lambda)$ depends on the product of an angle independent function $S_0(\lambda)$ and a wavelength independent scattering function of the object $g(\beta)$, particularly wherein $g(\beta)$ is different from the scattering function of the calibration target $f(\beta)$.

7. The spectrometer device (110) according to any one of the preceding claims, wherein the item of reflectance information classifies the object as
an object (112) of a third type, particularly having diffuse reflection properties, more particularly following the Lambert's cosine law, if the detector signal $S_\beta(\lambda)$ for a wavelength $\lambda$ is independent of an angle of incidence $\alpha$ of the illumination light (118) that is incident on the sample interface (130) and propagating to the object (112) and is proportional to the cosine of the angle of incidence $\beta$.

8. The spectrometer device (110) according to any one of the preceding claims, wherein the at least two photosensitive detectors (122) are designated to receive detection light (119) reflected and/or scattered by the object (112).

9. The spectrometer device (110) according to any one of the four preceding claims, wherein the spectrometer device (110) further comprises a signal device designated for indicating the item of reflectance information to a user, wherein the signal device is further designated to indicate to a user to repeat the measurement when a classification of the object (112) into at least one type failed.

10. The spectrometer device (110) according to any one of the preceding claims, wherein the at least one evaluation unit (140) is further configured to determine at least one item of spectral information on the object (112).

11. The spectrometer device (110) according to any one of the three preceding claims,
wherein the at least one evaluation unit (140) is further configured to provide the spectral information on the object (112) when the item of reflectance information indicates a reflectance type of the object (112).

12. A method for determining reflectance information on the object (112), wherein the method comprising using a spectrometer device (110) according to any one of the preceding claims, wherein a reflectance information on the object (112) is determined by considering the detector signals of the at least two photosensitive detectors (122).

13. A computer program comprising instructions which, when the program is executed by the spectrometer device (110) according to any one of the preceding claims referring to a spectrometer device (110) cause the evaluation unit (140) of the spectrometer device (110) to determine at least one item of reflectance information about the at least one measurement object (112) by considering a detector signal.

14. A computer-readable storage medium comprising instructions which, when the program is executed by the spectrometer device (110) according to any one of the preceding claims referring to a spectrometer device (110) cause the evaluation unit (140) of the spectrometer device (110) to determine at least one item of reflectance information about the at least one measurement object (112) by considering a detector signal.

15. A non-transient computer-readable medium including instructions that, when executed by one or more processors of an evaluation unit (140) of a spectrometer device (110) according to any one of the preceding claims referring to a spectrometer device (110) cause one or more processors to determine at least one item of reflectance information about the at least one measurement object (112) by considering a detector signal.

# FIG.1

# FIG.2

FIG.3

Europäisches
Patentamt

European
Patent Office

Office européen
des brevets

# EUROPEAN SEARCH REPORT

Application Number

EP 23 18 4736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/245632 A1 (NISPER JON K [US] ET AL) 2 November 2006 (2006-11-02) * figures 1-3, 9-18 * * paragraphs [0006], [0024] - [0034], [0046] - [0055] * * table 1 * | 1-15 | INV. G01N21/47 G01N21/31 A61B5/00 G01J3/02 |
| X | US 2019/120764 A1 (ISHII TOSHIYUKI [JP] ET AL) 25 April 2019 (2019-04-25) * figures 1A-3, 6-14 * * paragraphs [0042] - [0109] * | 1-15 | ADD. G01N21/27 |
| X | US 2004/051862 A1 (ALCOCK ROBIN DANIEL [GB] ET AL) 18 March 2004 (2004-03-18) * figures 5, 6, 9, 12-15 * * paragraphs [0192] - [0230] * | 1-15 | |
| X | US 2018/024005 A1 (EHBETS PETER [CH] ET AL) 25 January 2018 (2018-01-25) * figures 1-4, 14-25 * * paragraphs [0097] - [0101], [0144] - [0167] * | 1-15 | |
| A | BERNAD B ET AL: "Deviation of white diffuse reflectance standards from perfect reflecting diffuser at visible and near-infrared spectral ranges", METROLOGIA, INSTITUTE OF PHYSICS PUBLISHING, BRISTOL, GB, vol. 56, no. 5, 9 August 2019 (2019-08-09) , page 55005, XP020343197, ISSN: 0026-1394, DOI: 10.1088/1681-7575/AB3351 [retrieved on 2019-08-09] * the whole document * | 1-15 | TECHNICAL FIELDS SEARCHED (IPC) G01N G01J A61B |
| A | WO 2023/052608 A1 (TRINAMIX GMBH [DE]) 6 April 2023 (2023-04-06) * figure 1 * | 1-15 | |

The present search report has been drawn up for all claims

| Place of search | Date of completion of the search | Examiner |
|---|---|---|
| Munich | 30 November 2023 | Sauerer, Christof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    .................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 18 4736**

This annex lists the patent family members relating to the patent documents cited in the above-mentioned European search report.
The members are as contained in the European Patent Office EDP file on
The European Patent Office is in no way liable for these particulars which are merely given for the purpose of information.

**30-11-2023**

| Patent document cited in search report | | | Publication date | Patent family member(s) | | | Publication date |
|---|---|---|---|---|---|---|---|
| US 2006245632 | A1 | | 02-11-2006 | CN | 101184986 | A | 21-05-2008 |
| | | | | EP | 1880196 | A1 | 23-01-2008 |
| | | | | JP | 4846787 | B2 | 28-12-2011 |
| | | | | JP | 2008539439 | A | 13-11-2008 |
| | | | | US | 2006245632 | A1 | 02-11-2006 |
| | | | | WO | 2006116386 | A1 | 02-11-2006 |
| US 2019120764 | A1 | | 25-04-2019 | JP | 6894672 | B2 | 30-06-2021 |
| | | | | JP | 2017207367 | A | 24-11-2017 |
| | | | | US | 2019120764 | A1 | 25-04-2019 |
| | | | | WO | 2017199691 | A1 | 23-11-2017 |
| US 2004051862 | A1 | | 18-03-2004 | AU | 9403501 | A | 22-04-2002 |
| | | | | CA | 2423689 | A1 | 18-04-2002 |
| | | | | EP | 1325477 | A2 | 09-07-2003 |
| | | | | EP | 1857985 | A1 | 21-11-2007 |
| | | | | EP | 1867977 | A1 | 19-12-2007 |
| | | | | GB | 2373324 | A | 18-09-2002 |
| | | | | US | 2004051862 | A1 | 18-03-2004 |
| | | | | WO | 0231780 | A2 | 18-04-2002 |
| US 2018024005 | A1 | | 25-01-2018 | CN | 103808666 | A | 21-05-2014 |
| | | | | CN | 108051374 | A | 18-05-2018 |
| | | | | EP | 2728342 | A1 | 07-05-2014 |
| | | | | EP | 2930494 | A1 | 14-10-2015 |
| | | | | KR | 20140058389 | A | 14-05-2014 |
| | | | | US | 2014152990 | A1 | 05-06-2014 |
| | | | | US | 2018024005 | A1 | 25-01-2018 |
| | | | | US | 2019049304 | A1 | 14-02-2019 |
| WO 2023052608 | A1 | | 06-04-2023 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017222618 A